# EUROPEAN PATENT SPECIFICATION

(11) **EP 4 292 519 B1**
(45) Date of publication and mention of the grant of the patent: **29.07.2026**
(21) Application number: 22778885.8
(22) Date of filing: 28.03.2022
(51) Int. Cl.: A61B 5/00, A61B 5/11, G06F 3/01, G06V 40/20, G08B 21/24

(54) **METHOD, APPARATUS, AND SYSTEM FOR DETECTING WEARING TIGHTNESS OF A WEARABLE DEVICE**
VERFAHREN, VORRICHTUNG UND SYSTEM ZUR ERMITTLUNG DER TRAGEFESTIGKEIT EINER TRAGBAREN VORRICHTUNG
PROCÉDÉ, APPAREIL ET SYSTÈME DE DÉTECTION DU DEGRÉ DE SERRAGE D'UN DISPOSITIF PORTABLE

(30) Priority: 31.03.2021 CN 202110347924
(43) Date of publication of application: 20.12.2023
(73) Proprietor: Huawei Technologies Co., Ltd., Shenzhen, Guangdong 518129 (CN)
(72) Inventor: GUO, Jianhua, Shenzhen, Guangdong 518129 (CN); XU, Teng, Shenzhen, Guangdong 518129 (CN); SUN, Yu, Shenzhen, Guangdong 518129 (CN)
(74) Representative: Maiwald GmbH
(86) International application number: PCT/CN2022/083467
(87) International publication number: WO 2022/206703

(56) References cited:
- EP-A1- 3 064 129
- WO-A1-2016/102416
- CN-A- 104 352 227
- CN-A- 106 441 413
- CN-A- 109 150 220

## Description

### TECHNICAL FIELD

Embodiments of the present invention relate to the field of electronic device technologies, and in particular, to a method, apparatus, and system for detecting wearing tightness of a wearable device.

### BACKGROUND

EP3064129A1 describes that a method includes determining whether the electronic device is attached on a human body and controlling the electronic device based on information regarding at least one of a body condition of a user, the body condition measured when the electronic device is attached, the user wearing the electronic device a position where the electronic device is attached or detached, or when the electronic device is attached or detached.

CN109150220A describes a method for adjusting the tightness degree of a wearable device. It is described that when the wearable device is in a wearing state, the working modes of the wearable device are determined, the working modes of the wearable device include at least two types, target tightness degree parameters corresponding to a current working mode of the wearable device is determined according to the preset correspondence relationship between the working modes and tightness degree parameters, wherein the tightness degree parameters are used for characterizing the wearing tightness degree of the wearable device, and the wearable device is adjusted according to the target tightness degree parameters.

CN106441413A describes a method and a device for detecting wearing tightness of a wearable device. It is described that the method comprises the steps of obtaining wearing state data of the wearable device on the condition that a wearing state of the wearable device is detected; determining a wearing tightness degree of the wearable device according to the wearing state data; and reminding a user according to the tightness degree.

With development of technologies, wearable devices are widely applied to people's life, work, and study. For example, the wearable device may detect a health status and a movement status of a user. When the user wears the wearable device loosely, data collected by the wearable device is inaccurate, which causes the wearable device to incorrectly evaluate the health status or the movement status of the user, and affects detection performance of the wearable device. Therefore, how to detect the wearing tightness of the wearable device is a technical problem to be resolved in this field.

### SUMMARY

Embodiments of this application provide a method, apparatus, and system for detecting wearing tightness of a wearable device, to detect the wearing tightness of the wearable device, and ensure detection performance of the wearable device.

The invention and its scope of protection is defined by the appended independent claims, where the dependent claims define embodiments of the invention. The following aspects and implementations of the summary provide examples of how technical subject matters can be combined.

According to a first aspect, an embodiment of this application provides a method for detecting wearing tightness of a wearable device. An execution body of the method may be an electronic device (for example, the wearable device), or may be a component (for example, a chip, a chip system, or a processor) located in an electronic device. The following uses an example in which the execution body is the electronic device for description. The method includes: collecting movement data of a user when it is detected that the wearable device is in a worn state; identifying action data of a first action in the movement data of the user; and determining the wearing tightness of the wearable device corresponding to the action data of the first action.

The movement data of the user may include an acceleration and/or an angular velocity. The wearing tightness of the wearable device may be understood as a tightness degree (for example, that the wearable device is worn normally, that the wearable device is worn loosely, and that the wearable device is worn tightly), or may be understood as a specific value.

The first action is an action of the user in a movement process. The first action corresponds to a position where the wearable device is worn, and when the wearable device is worn on an arm or a wrist of the user, the first action may include an arm swinging action, and the like. When the wearable device is worn on an ankle or a running shoe of the user, the first action may include a jump action, a kicking action, and the like.

In this way, in this embodiment of this application, the wearing tightness of the wearable device can be determined based on the movement data of the user. This can avoid inaccurate movement data collection, make movement data collection more reliable, and lay a foundation for providing more professional guidance for the user.

In a possible implementation, the determining the wearing tightness of the wearable device corresponding to the action data of the first action may be specifically: determining the wearing tightness of the wearable device based on the action data of the first action and a preset correspondence between the movement data and the wearing tightness.

The preset correspondence between the movement data and the wearing tightness may be understood as follows: In a first case, there is a correspondence between one piece of movement data and the wearing tightness. In other words, a value of the movement data is in a one-to-one correspondence with the wearing tightness. For example, if the movement data is an acceleration, and the acceleration value is 30 m/s², the movement data corresponds to a wearing tightness that the wearable device is worn normally. In a second case, there is a correspondence between a group of movement data and the wearing tightness. In other words, a value range of the movement data is in a correspondence with the wearing tightness. For example, if the movement data is an acceleration, and the value range of the acceleration is 30 to 50 m/s², the movement data corresponds to a wearing tightness that the wearable device is worn normally.

In this embodiment of this application, the wearable device may determine the wearing tightness of the wearable device based on the movement data of the user, and may detect the wearing tightness of the wearable device without an additional hardware apparatus driving an action of the wearable device. This can effectively avoid a problem of inaccurate collection of the action data, so that collection of the action data is more reliable, and a foundation for providing more professional guidance for the user is laid.

In a possible implementation, the determining the wearing tightness of the wearable device based on the action data of the first action and a preset correspondence between the movement data and the wearing tightness may be specifically: identifying action data of a first action node in the action data of the first action; determining first feature data corresponding to the first action node based on the action data of the first action node; and determining the wearing tightness of the wearable device based on the first feature data and the preset correspondence between the movement data and the wearing tightness.

In a possible implementation, the first feature data includes at least one of a wave peak quantity of an acceleration waveform, a wave peak quantity of an angular velocity waveform, a wave trough quantity of the acceleration waveform, a wave trough quantity of the angular velocity waveform, a peak value of the acceleration waveform, a peak value of the angular velocity waveform, an acceleration average value, an angular velocity average value, a gradient of the acceleration waveform, and a gradient of the angular velocity waveform.

The acceleration average value may be understood as an acceleration average value in a preset time period. The angular velocity average value may be understood as an angular velocity average value in a preset time period. The gradient of the acceleration waveform may be understood as a slope of a tangent of a preset position in the acceleration waveform. The gradient of the angular velocity waveform may be understood as a slope of a tangent of a preset position in the angular velocity waveform.

In a possible implementation, the determining the wearing tightness of the wearable device corresponding to the action data of the first action may be specifically: determining, based on the action data of the first action and a wearing tightness model, the wearing tightness of the wearable device corresponding to the action data of the first action. The action data of the first action is used as an input of the wearing tightness model, and the wearing tightness of the wearable device is used as an output of the wearing tightness model.

In a possible implementation, after the determining the wearing tightness of the wearable device corresponding to the action data of the first action, the method for detecting the wearing tightness of the wearable device provided in embodiments of this application may further include: generating prompt information based on the wearing tightness of the wearable device, where the prompt information presents a detection result of the wearing tightness of the wearable device in a preset presentation form. The preset presentation form includes one or a combination of the following: a sound presentation form, a text presentation form, a vibration presentation form, and a light presentation form.

In this embodiment of this application, the detection result is notified to the user in different forms, so that a phenomenon that the wearable device of the user easily falls off and is lost in a movement process of the user can be effectively avoided, and a risk of collision or accidentally collision of the user in the movement process can be avoided.

In a possible implementation, the wearing tightness of the wearable device includes: the wearable device is worn normally; the wearable device is worn loosely; and the wearable device is worn tightly.

In some embodiments, the method for detecting the wearing tightness of the wearable device provided in this embodiment of this application further includes: determining wearing tightness of the wearable device at each moment in first duration; determining second duration in which the wearable device is worn loosely based on the wearing tightness of the wearable device at each moment in the first duration; and calculating second movement energy of the user in the first duration based on the first duration, the second duration, and first movement energy calculated by the wearable device. The first movement energy is obtained through calculation when a wearing status of the wearable device in the first duration is normal or tight.

Both the first movement energy and the second movement energy may be understood as movement energy consumption of the user, for example, heat consumed by the user during movement, and calories burnt by the user during the movement.

The second duration may be continuous duration, or may be a sum of a plurality of discontinuous periods of duration. To be specific, the second duration may be a continuous period of duration in which the wearable device is worn loosely, or may be a sum of a plurality of discontinuous periods of duration in which the wearable device is worn loosely.

Therefore, in this embodiment of this application, the wearable device may evaluate movement energy of the user in the second duration. When the wearable device is worn loosely, the wearable device may determine actual movement energy of a movement of the user in the second duration based on a wearing looseness time proportion and evaluated movement energy, so that the movement data of the user is more reliable, to remind the user to pay attention to adjusting the wearing tightness of the wearable device during next running.

In a possible implementation, the first action includes a jump action, and the first action node includes: a foot leaves the ground and/or the foot touches the ground; or the first action includes an arm swinging action, and the first action node includes: an arm swings on a front side of a body at a specified position and/or the arm swings on a rear side of the body at a specified position.

In a possible implementation, the wearable device is configured to be worn on the foot or a shoe of the user.

According to a second aspect, an embodiment of this application provides an apparatus for detecting wearing tightness of a wearable device. The apparatus includes: a collection unit, configured to collect movement data of a user when it is detected that the wearable device is in a worn state; an identification unit, configured to identify action data of a first action in the movement data of the user; and a first determining unit, configured to determine the wearing tightness of the wearable device corresponding to the action data of the first action. In this way, in this embodiment of this application, the wearing tightness of the wearable device can be determined based on the movement data of the user. This can avoid inaccurate movement data collection, make movement data collection more reliable, and lay a foundation for providing more professional guidance for the user.

In a possible implementation, the first determining unit is further configured to determine the wearing tightness of the wearable device based on the action data of the first action and a preset correspondence between the movement data and the wearing tightness.

In a possible implementation, the first determining unit is further configured to: identify action data of a first action node in the action data of the first action; determine first feature data corresponding to the first action node based on the action data of the first action node; and determine the wearing tightness of the wearable device based on the first feature data and the preset correspondence between the movement data and the wearing tightness.

In a possible implementation, the first feature data includes at least one of a wave peak quantity of an acceleration waveform, a wave peak quantity of an angular velocity waveform, a wave trough quantity of the acceleration waveform, a wave trough quantity of the angular velocity waveform, a peak value of the acceleration waveform, a peak value of the angular velocity waveform, an acceleration average value, an angular velocity average value, a gradient of the acceleration waveform, and a gradient of the angular velocity waveform.

In a possible implementation, the first determining unit is further configured to: determine, based on the action data of the first action and a wearing tightness model, the wearing tightness of the wearable device corresponding to the action data of the first action. The action data of the first action is used as an input of the wearing tightness model, and the wearing tightness of the wearable device is used as an output of the wearing tightness model.

In some embodiments, the apparatus for detecting the wearing tightness of the wearable device provided in this embodiment of this application further includes a generation unit, configured to generate prompt information based on the wearing tightness of the wearable device, where the prompt information presents a detection result of the wearing tightness of the wearable device in a preset presentation form. The preset presentation form includes one or a combination of the following: a sound presentation form, a text presentation form, a vibration presentation form, and a light presentation form.

In this embodiment of this application, the detection result is notified to the user in different forms, so that a phenomenon that the wearable device of the user easily falls off and is lost in a movement process of the user can be effectively avoided, and a risk of collision or accidentally collision of the user in the movement process can be avoided.

In a possible implementation, the wearing tightness of the wearable device includes: the wearable device is worn normally; the wearable device is worn loosely; and the wearable device is worn tightly.

In some embodiments, the apparatus for detecting the wearing tightness of the wearable device provided in this embodiment of this application further includes: a second determining unit, configured to determine wearing tightness of the wearable device at each moment in first duration; a third determining unit, configured to determine second duration in which the wearable device is worn loosely based on the wearing tightness of the wearable device at each moment in the first duration; and a calculation unit, configured to calculate second movement energy of the user in the first duration based on the first duration, the second duration, and first movement energy calculated by the wearable device. The first movement energy is obtained through calculation when a wearing status of the wearable device in the first duration is normal or tight.

Therefore, in this embodiment of this application, the wearable device may evaluate movement energy of the user in the second duration. When the wearable device is worn loosely, the wearable device may determine actual movement energy of a movement of the user in the second duration based on a wearing looseness time proportion and evaluated movement energy, so that the movement data of the user is more reliable, to remind the user to pay attention to adjusting the wearing tightness of the wearable device during next running.

In a possible implementation, the first action includes a jump action, and the first action node includes: a foot leaves the ground and/or the foot touches the ground; or the first action includes an arm swinging action, and the first action node includes: an arm swings on a front side of a body at a specified position and/or the arm swings on a rear side of the body at a specified position.

In a possible implementation, the wearable device is configured to be worn on a foot or a shoe of the user.

It may be understood that the apparatus includes a function of implementing any method according to the foregoing aspects and the possible implementations. The function may be implemented by hardware, or may be implemented by hardware executing corresponding software. The hardware or the software includes at least one module or unit corresponding to the foregoing function.

According to a third aspect, an embodiment of this application provides a system for detecting wearing tightness of a wearable device. The system includes: the wearable device, configured to: collect movement data of a user when it is detected that the wearable device is in a worn state; identify action data of a first action in the movement data of the user; and determine the wearing tightness of the wearable device corresponding to the action data of the first action, and send the wearing tightness of the wearable device to a non-wearable device; and the non-wearable device, configured to: receive the wearing tightness of the wearable device, and generate prompt information based on the wearing tightness of the wearable device, where the prompt information is for notifying the user of a detection result of the wearing tightness of the wearable device.

In this embodiment of this application, a function of detecting the wearing tightness of the wearable device is extended to a plurality of devices. Through a cooperative operation between the plurality of devices, a problem of the wearing tightness of the user is resolved, and experience of linkage between a plurality of devices of the user is improved.

According to a fourth aspect, an embodiment of this application provides a system for detecting wearing tightness of a wearable device. The system includes: the wearable device, configured to: collect movement data of a user when it is detected that the wearable device is in a worn state; and send the collected movement data to a non-wearable device; and the non-wearable device, configured to: receive the movement data sent by the wearable device; identify action data of a first action in the movement data; and determine the wearing tightness of the wearable device corresponding to the action data of the first action.

In a possible implementation, the non-wearable device is further configured to generate prompt information based on the wearing tightness of the wearable device, where the prompt information is for notifying the user of a detection result of the wearing tightness of the wearable device.

In this embodiment of this application, a function of detecting the wearing tightness of the wearable device and a prompt function are extended to a plurality of devices. Through a cooperative operation between the plurality of devices, a problem of the wearing tightness of the user is resolved, and experience of linkage between a plurality of devices of the user is improved.

According to a fifth aspect, a computer-readable storage medium is provided, including computer instructions. When the computer instructions are run on a terminal, the terminal is enabled to perform the method according to any one of the foregoing aspects and the possible implementations of the foregoing aspects.

According to a sixth aspect, a computer program product is provided. When the computer program product is run on a computer, the computer is enabled to perform the method according to any one of the foregoing aspects and the possible implementations of the foregoing aspects.

According to a seventh aspect, a chip system is provided, including a processor. When the processor executes instructions, the processor performs the method according to any one of the foregoing aspects and the possible implementations of the foregoing aspects.

For specific implementations and corresponding technical effects of the embodiments in the second aspect to the seventh aspect, refer to the specific implementations and technical effects of the first aspect.

### BRIEF DESCRIPTION OF DRAWINGS

To explain technical solutions in embodiments of this application more clearly, the following briefly describes accompanying drawings used for describing the embodiments or a current technology. It is clear that the accompanying drawings in the following descriptions show merely some embodiments of this application, and a person of ordinary skill in the art may still derive other drawings from the accompanying drawings without creative efforts.
FIG. 1 is a schematic diagram of a structure of a wearable device according to an embodiment of this application;
FIG. 2 is a schematic flowchart of a method for detecting wearing tightness of a wearable device according to an embodiment of this application;
FIG. 3 is a schematic diagram of an application scenario of a method for detecting wearing tightness of a wearable device according to an embodiment of this application;
FIG. 4 is a schematic diagram of an application scenario of a method for detecting wearing tightness of a wearable device according to an embodiment of this application;
FIG. 5 is a schematic diagram of an application scenario of a method for detecting wearing tightness of a wearable device according to an embodiment of this application;
FIG. 6a is a schematic diagram of an application scenario of a method for detecting wearing tightness of a wearable device according to an embodiment of this application;
FIG. 6b is a schematic diagram of an application scenario of a method for detecting wearing tightness of a wearable device according to an embodiment of this application;
FIG. 7 is a schematic flowchart of a method for detecting wearing tightness of a wearable device in an application scenario according to an embodiment of this application;
FIG. 8 is a schematic flowchart of a method for detecting wearing tightness of a wearable device in an application scenario according to an embodiment of this application;
FIG. 9 is a schematic flowchart of a method for detecting wearing tightness of a wearable device in an application scenario according to an embodiment of this application;
FIG. 10 is a schematic flowchart of a method for detecting wearing tightness of a wearable device according to an embodiment of this application;
FIG. 11 is a schematic flowchart of a method for detecting wearing tightness of a wearable device according to an embodiment of this application; and
FIG. 12 is a schematic diagram of a structure of an apparatus for detecting wearing tightness of a wearable device according to an embodiment of this application.

### DESCRIPTION OF EMBODIMENTS

In descriptions of embodiments of this application, unless otherwise specified, "/" means "or". For example, A/B may represent A or B. In this specification, "and/or" describes only an association relationship between associated objects and represents that three relationships may exist. For example, A and/or B may represent the following three cases: Only A exists, both A and B exist, and only B exists.

The terms "first" and "second" mentioned below are merely intended for a purpose of description, and shall not be understood as an indication or implication of relative importance or implicit indication of the quantity of indicated technical features. Therefore, a feature limited by "first" or "second" may explicitly or implicitly include one or more features. In the descriptions of embodiments of this application, unless otherwise specified, "a plurality of" means two or more than two.

In embodiments of this application, the word "example" or "for example" is used for representing giving an example, an illustration, or descriptions. Any embodiment or design scheme described as an "example" or "for example" in embodiments of this application should not be explained as being more preferred or having more advantages than another embodiment or design scheme. Exactly, use of the word "example", "for example", or the like is intended to present a related concept in a specific manner.

With development of technologies, wearable devices are widely applied to people's life, work, and study. For example, the wearable device may detect a health status and movement status of a user, and the like. When the wearable device is worn loosely on the user, data collected by the wearable device is inaccurate, which causes the wearable device to incorrectly evaluate the health status or the movement status of the user, and affects detection performance of the wearable device.

To ensure the detection performance of the wearable device, the following manner may be used for detecting wearing tightness of the wearable device. A pressure sensor disposed on a bottom shell of the wearable device detects a pressure value on the bottom shell of the wearable device, and determines the wearing tightness of the wearable device based on the detected pressure value. This resolves a problem of detecting the wearing tightness of the wearable device, and improves comfort of the user and accuracy of the detection performance of the wearable device.

An embodiment of this application provides another method for detecting wearing tightness of a wearable device: collecting movement data of a user when it is detected that the wearable device is in a worn state; identifying action data of a first action in the movement data of the user; and determining the wearing tightness of the wearable device corresponding to the action data of the first action. In this way, in this embodiment of this application, the wearing tightness of the wearable device can be determined based on the movement data of the user. This can avoid inaccurate movement data collection, make movement data collection more reliable, and lay a foundation for providing more professional guidance for the user.

Further, the determining the wearing tightness of the wearable device corresponding to the action data of the first action may be specifically implemented in the following manners. Manner 1: Determine the wearing tightness of the wearable device based on the action data of the first action and a correspondence between the movement data and the wearing tightness. For example, first feature data corresponding to first action node of the first action of the user is determined based on the action data of the first action. The wearing tightness of the wearable device is determined based on a correspondence between feature data and the wearing tightness and the first feature data corresponding to the first action node. Manner 2: Determine, based on the action data of the first action and a wearing tightness model, the wearing tightness of the wearable device corresponding to the action data of the first action. The action data of the first action is used as an input of the wearing tightness model, the wearing tightness of the wearable device is used as an output of the wearing tightness model, and the wearing tightness model is obtained based on the movement data and the wearing tightness of the wearable device through training.

Further, the method for detecting the wearing tightness of the wearable device provided in this embodiment of this application further includes: generating prompt information based on the wearing tightness of the wearable device, where the prompt information presents a detection result of the wearing tightness of the wearable device to the user in a preset presentation form. The preset presentation form includes one or a combination of the following: a sound presentation form, a text presentation form, a vibration presentation form, and a light presentation form. In this way, the detection result can be effectively and intuitively displayed to the user, to achieve a timely reminding function.

There may be one or more execution bodies of the method for detecting the wearing tightness of the wearable device provided in this embodiment of this application.

When there is one execution body of the method for detecting the wearing tightness of the wearable device, the execution body of the method for detecting the wearing tightness of the wearable device may be a wearable device.

When there are at least two execution bodies of the method for detecting the wearing tightness of the wearable device, the execution bodies of the method for detecting the wearing tightness of the wearable device may include a wearable device and a non-wearable device, where the non-wearable device may be a device such as a mobile phone, a computer, a headset, a watch, or a band. A cooperative operation between the wearable device and the non-wearable device may be implemented in a plurality of manners. For example, the manners may be as follows.

Manner 1: When detecting that the wearable device is in the worn state, the wearable device collects the movement data of the user; identify the action data of the first action in the movement data of the user; and determine the wearing tightness of the wearable device corresponding to the action data of the first action, and send the wearing tightness of the wearable device to the non-wearable device. Specifically, when detecting that the wearable device is in the worn state, the wearable device collects the movement data of the user. The wearable device identifies the action data of the first action from the collected movement data of the user, where the first action includes at least one action node. The wearable device determines the first feature data corresponding to the first action node of the first action of the user. The wearable device determines the wearing tightness of the wearable device based on a correspondence between movement data (for example, first standard data of the first action node) stored in a standard database and the wearing tightness and the first feature data corresponding to the first action node. The wearable device sends the wearing tightness of the wearable device to the non-wearable device. The non-wearable device receives the wearing tightness of the wearable device, and generates the prompt information based on the wearing tightness of the wearable device to notify the user of the detection result of the wearing tightness of the wearable device.

Manner 2: When detecting that the wearable device is in the worn state, the wearable device collects the movement data of the user; the wearable device sends the collected movement data to the non-wearable device; and the non-wearable device receives the movement data sent by the wearable device; identifies the action data of the first action in the movement data; and determines the wearing tightness of the wearable device corresponding to the action data of the first action. Specifically, the non-wearable device identifies the action data of the first action from the movement data, where the first action includes the at least one action node. The non-wearable device determines the first feature data corresponding to the first action node of the first action of the user. The non-wearable device determines the wearing tightness of the wearable device based on a correspondence between movement data (for example, first standard data of the first action node) stored in a standard database and the wearing tightness and the first feature data corresponding to the first action node. Further, the non-wearable device generates the prompt information based on the wearing tightness of the wearable device to notify the user of the detection result of the wearing tightness of the wearable device. There may be another manner of the cooperative operation between the wearable device and the non-wearable device.

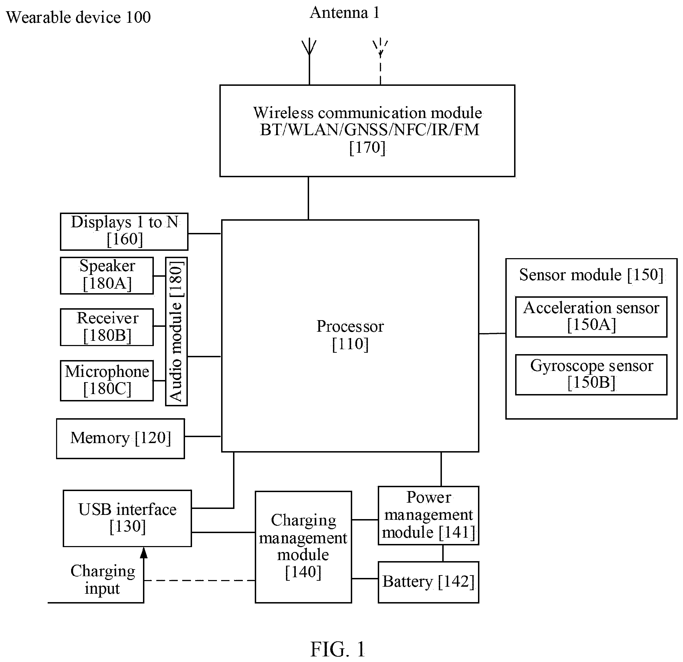

The following describes structures of the wearable device or the non-wearable device. The structure of the wearable device may be the same as that of the non-wearable device. In this embodiment of this application, an example in which the wearable device and the non-wearable device have a same structure is used for description. FIG. 1 is a schematic diagram of a structure of a wearable device 100.

The wearable device 100 may include a processor 110, a memory 120, a universal serial bus (universal serial bus, USB) interface 130, a charging management module 140, a power management module 141, a battery 142, a sensor module 150, a display 160, an antenna 1, a wireless communication module 170, an audio module 180, and the like. The sensor module 150 may include an acceleration sensor 150A, a gyroscope sensor 150B, and the like. The audio module 180 may include a speaker 180A, a receiver 180B, a microphone 180C, and the like.

It may be understood that the structure shown in this embodiment of the present invention does not constitute a specific limitation on the wearable device 100. In some other embodiments of this application, the wearable device 100 may include more or fewer components than those shown in the figure, or some components may be combined, or some components may be split, or have a different component arrangement. The components shown in the figure may be implemented by hardware, software, or a combination of the software and the hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may generate an operation control signal based on an instruction operation code and a time sequence signal, to complete control of instruction reading and instruction execution.

A memory may be further disposed in the processor 110, and is configured to store instructions and data. In some embodiments, the memory in the processor 110 is a cache memory. The memory may store instructions or data that has just been used or cyclically used by the processor 110. If the processor 110 needs to use the instructions or the data again, the processor may directly invoke the instructions or the data from the memory. This avoids repeated access, reduces waiting time of the processor 110, and improves system efficiency.

In some embodiments, the processor 110 may include one or more interfaces. The interfaces may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

It may be understood that an interface connection relationship between the modules that is shown in this embodiment of the present invention is merely an example for description, and does not constitute a limitation on the structure of the wearable device 100. In some other embodiments of this application, the wearable device 100 may also use an interface connection manner different from that in the foregoing embodiment, or a combination of a plurality of interface connection manners.

The memory 120 may be configured to store computer-executable program code, where the executable program code includes instructions. The memory 120 may include a program storage area and a data storage area. The program storage area may store an operating system, an application required by at least one function (for example, a voice playing function or an image playing function), and the like. The data storage area may store data (such as audio data and an address book) created during use of the wearable device 100, and the like. In addition, the memory 120 may include a high-speed random access memory, and may further include a non-volatile memory, for example, at least one magnetic disk storage device, a flash memory, or a universal flash storage (universal flash storage, UFS). The processor 110 runs the instructions stored in the memory 120 and/or the instructions stored in the memory provided in the processor, to perform various function applications and data processing of the wearable device 100.

The charging management module 140 is configured to receive charging input from a charger. The charger may be a wireless charger or a wired charger. In some embodiments of wired charging, the charging management module 140 may receive charging input of the wired charger through the USB interface 130. In some embodiments of wireless charging, the charging management module 140 may receive wireless charging input through a wireless charging coil of the wearable device 100. The charging management module 140 supplies power to the wearable device through the power management module 141 while charging the battery 142.

The power management module 141 is configured to connect to the battery 142, the charging management module 140, and the processor 110. The power management module 141 receives input of the battery 142 and/or input of the charging management module 140, and supplies power to the processor 110, the memory 120, the display 160, the wireless communication module 170, and the like. The power management module 141 may be further configured to: monitor parameters such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same component.

The acceleration sensor 150A may detect accelerations in various directions (usually on three axes) of the wearable device 100. When the wearable device 100 is still, a magnitude and a direction of gravity may be detected. The acceleration sensor 150A may be further configured to identify a posture of the wearable device, and is used in an application such as switching between a landscape mode and a portrait mode and a pedometer.

The gyroscope sensor 150B may be configured to determine a movement posture of the wearable device 100. In some embodiments, angular velocities of the wearable device 100 around three axes (namely, axes x, y, and z) may be determined through the gyroscope sensor 150B. The gyroscope sensor 150B may be configured to implement image stabilization during photographing. For example, when a shutter is pressed, the gyroscope sensor 150B detects an angle of shaking of the wearable device 100, calculates a distance to be compensated by the lens module based on the angle, and allows a lens to cancel the shaking of the wearable device 100 through a reverse movement, to implement the image stabilization. The gyroscope sensor 150B may also be used in a navigation scenario and a somatic game scenario.

A wireless communication function of the wearable device 100 may be implemented through the antenna 1, the wireless communication module 170, the modem processor, the baseband processor, and the like.

The antenna 1 is configured to transmit and receive an electromagnetic wave signal. Each antenna in the wearable device 100 may be configured to cover one or more communication bands. Different antennas may be further multiplexed, to improve antenna utilization. In some other embodiments, the antenna may be used in combination with a tuning switch.

The wearable device 100 may implement a display function through the GPU, the display 160, the application processor, and the like. The GPU is a microprocessor for image processing, and is connected to the display 160 and the application processor. The GPU is configured to: perform mathematical and geometric computation, and render an image. The processor 110 may include one or more GPUs, which execute program instructions to generate or change display information.

The display 160 is configured to: display an image, a video, and the like. The display 160 includes a display panel. The display panel may be a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flexible light-emitting diode, FLED), a mini-LED, a micro-LED, a micro-OLED, a quantum dot light emitting diode (quantum dot light emitting diode, QLED), or the like. In some embodiments, the wearable device 100 may include one or N displays 160, where N is a positive integer greater than 1.

The wireless communication module 170 may provide a wireless communication solution that is applied to the wearable device 100 and that includes a wireless local area network (wireless local area network, WLAN) (such as a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, and the like. The wireless communication module 170 may be one or more components integrating at least one communication processor module. The wireless communication module 170 receives an electromagnetic wave by the antenna 1, performs the frequency modulation and filtering processing on the electromagnetic wave signal, and sends a processed signal to the processor 110. The wireless communication module 170 may further receive a to-be-sent signal from the processor 110, perform the frequency modulation and amplification on the signal, and convert the signal into an electromagnetic wave for radiation through the antenna 1.

The audio module 180 is configured to convert digital audio information into an analog audio signal for output, and is also configured to convert analog audio input into a digital audio signal. The audio module 180 may be further configured to code and decode an audio signal. In some embodiments, the audio module 180 may be disposed in the processor 110, or some functional modules in the audio module 180 are disposed in the processor 110.

The speaker 180A, also referred to as a "loudspeaker", is configured to convert an audio electrical signal into a sound signal. The wearable device 100 may be used for listening to music or answering a call in a hands-free mode through the speaker 180A.

The receiver 180B, also referred to as an "earpiece", is configured to convert the audio electrical signal into the sound signal. When a call is answered or voice information is received through the wearable device 100, the voice may be received by placing the receiver 180B close to a human ear.

The microphone 180C, also referred to as a "mike" or a "mic", is configured to convert the sound signal into an electrical signal. When making a call or sending voice information, a user may make a sound near the microphone 180C through a human mouth of the user, to input the sound signal to the microphone 180C. At least one microphone 180C may be disposed in an electronic device 300. In some other embodiments, two microphones 180C may be disposed in the wearable device 100, to collect the sound signal and implement a noise reduction function. In some other embodiments, three, four, or more microphones 180C may further be disposed in the wearable device 100, to collect the sound signal, reduce noise, identify a sound source, implement a directional recording function, and the like.

It is clear that the wearable device 100 may further include another functional unit. This is not limited in this embodiment of this application.

The following uses an architecture shown in FIG. 1 as an example to describe the method for detecting wearing tightness of a wearable device provided in this embodiment of this application. Each unit in the following embodiment may have a component shown in FIG. 1. Details are not described again. It should be noted that, in this embodiment of this application, names of messages exchanged between devices, names of parameters in the messages, or the like are merely examples. Another name may also be used during a specific implementation. Generating (generate) in this embodiment of this application may also be understood as creating (create) or determining, and "including" in this embodiment of this application may also be understood as "carrying". This is uniformly described herein, and details are not specifically limited in this embodiment of this application.

As described above, there may be one or more execution bodies of the method for detecting the wearing tightness of the wearable device provided in this embodiment of this application. For different execution bodies, detailed descriptions are provided herein.

In a first case, when there is one execution body of the method for detecting the wearing tightness of the wearable device, the execution body of the method for detecting the wearing tightness of the wearable device may be a wearable device.

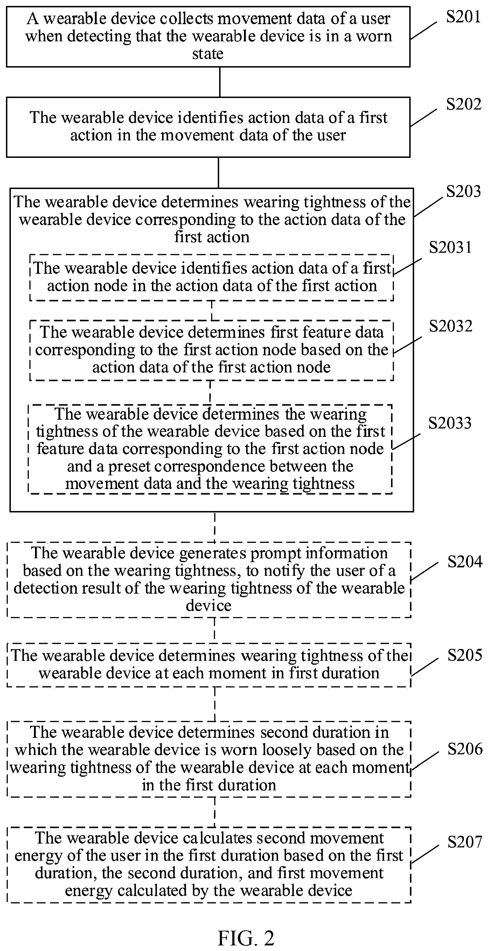

FIG. 2 is a schematic flowchart of a method for detecting wearing tightness of a wearable device according to an embodiment of this application. As shown in FIG. 2, an execution body of the method may be the wearable device, and the method may include the following steps.

S201: The wearable device collects movement data of a user when detecting that the wearable device is in a worn state.

For example, that the wearable device is in a worn state may mean that the wearable device may be worn on an arm or a wrist of the user, or the wearable device may be worn on an ankle or a running shoe of the user. Specifically, the wearable device is worn on the running shoe of the user. Specifically, the wearable device may be connected to the running shoe through a buckle, or it is clear that the wearable device may be connected in another attaching manner specifically. This is not specifically limited in this embodiment of this application. The wearable device may be worn on a vamp, an upper, or a shoelace of the running shoe. Specifically, the wearable device is worn on the shoelace of the user. It can be learned that a wearing tightness detection solution in a scenario of wearing the running shoe is added to the method for detecting the wearing tightness of the wearable device provided in this embodiment of this application.

For example, the wearable device may collect the movement data of the user in the following manners. Manner 1: An acceleration sensor on the wearable device collects an acceleration of a first body part. Manner 2: A gyroscope sensor on the wearable device collects an angular velocity of the first body part. Manner 3: The acceleration sensor on the wearable device collects the acceleration of the first body part, and the gyroscope sensor on the wearable device collects the angular velocity of the first body part. In the foregoing implementation, the first body part is related to a movement of the user.

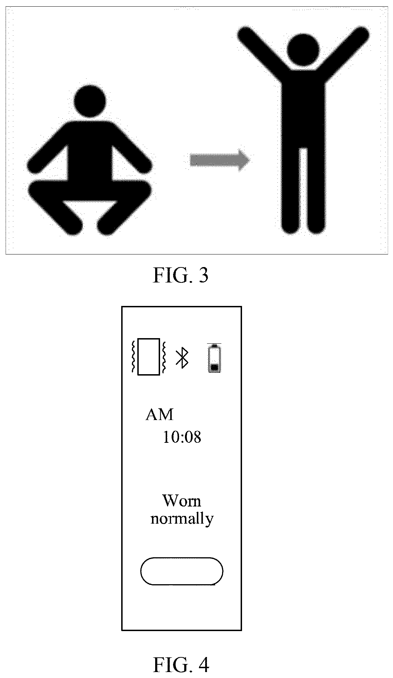

Example 1: FIG. 3 is a schematic diagram of an application scenario of a method for detecting wearing tightness of a wearable device according to an embodiment of this application. As shown in FIG. 3, an example in which a movement of a user is a bounce movement is used, and correspondingly, a first body part may be a foot of a human body. In this case, when the user performs the bounce, an acceleration sensor on the wearable device collects an acceleration of the foot of the user, and/or a gyroscope sensor on the wearable device collects an angular velocity of the foot of the user.

Example 2: An example in which the movement of the user is an arm swinging movement is used, and correspondingly, the first body part may be an arm of the human body. In this case, when the user performs the arm swinging movement, the acceleration sensor on the wearable device collects an acceleration of the arm of the user, and/or the gyroscope sensor on the wearable device collects an angular velocity of the arm of the user.

S202: The wearable device identifies action data of a first action in the movement data of the user.

The first action is an action of the user in a movement process. The first action corresponds to a position where the wearable device is worn, and when the wearable device is worn on the arm or a wrist of the user, the first action may include an arm swinging action, and the like. When the wearable device is worn on an ankle or a running shoe of the user, the first action may include a jump action, a kicking action, and the like. It is clear that the first action may include any action that can be completed by a human body. This is not specifically limited in this embodiment of this application.

The first action may include at least one action node. The action node may be understood as a node in which a body part of the user changes in a process in which the user performs an action. For example, in an example in which the first action is the jump action, the action node may include two action nodes, for example, a foot leaves the ground and the foot touches the ground. In an example in which the first action is the arm swinging action, the action node may include action nodes, for example, an arm swings on a front side of a body at a specified position and the arm swings on a rear side of the body at a specified position.

That the wearable device identifies action data of a first action in the movement data of the user may be specifically implemented as follows: The wearable device determines the action data of the first action based on collected real-time movement data of the user in the movement process, prestored actions, and standard data corresponding to the actions. In other words, the wearable device compares the collected movement data with the standard data of all the actions, and determines, from the collected movement data, first movement data similar to the standard data of the first action. The wearable device determines an action corresponding to the first movement data as the first action, and determines that the first movement data is the action data of the first action. To be specific, the wearable device compares the collected movement data with the standard data of all the actions, and determines the standard data of the first action that is close to the first movement data. The wearable device determines the first action as the action corresponding to the first movement data, and the first movement data is the action data of the first action.

S203: The wearable device determines the wearing tightness of the wearable device corresponding to the action data of the first action.

For example, S203 may be specifically implemented in the following manners.

Manner 1: The wearable device determines the wearing tightness of the wearable device based on the action data of the first action and a preset correspondence between the movement data and the wearing tightness. To be specific, the wearable device determines the wearing tightness of the wearable device based on the action data of the first action and a correspondence between standard data of the first action stored in a standard database and the wearing tightness.

The preset correspondence between the movement data and the wearing tightness may be understood as follows: In a first case, there is a correspondence between one piece of movement data and the wearing tightness. In other words, a value of the movement data is in a one-to-one correspondence with the wearing tightness. For example, if the movement data is an acceleration, and the acceleration value is 30 m/s², the movement data corresponds to a wearing tightness that the wearable device is worn normally. In a second case, there is a correspondence between a group of movement data and the wearing tightness, in other words, a value range of the movement data is in a correspondence with the wearing tightness. For example, if the movement data is an acceleration, and the value range of the acceleration is 30 to 50 m/s², the movement data corresponds to a wearing tightness that the wearable device is worn normally.

In addition, the first action may include at least one action node. To accurately determine the wearing tightness of the wearable device, the wearable device may determine action data of each action node based on the action data of the first action. An example is as follows:

S2031: The wearable device identifies action data of a first action node in the action data of the first action.

The first action node may be determined based on at least one of a sudden change of the movement data, an acceleration direction, and an angular velocity direction.

Specifically, it may be implemented as follows: The wearable device determines an action waveform formed by action data of the first body part in a process in which the user performs the first action. The wearable device determines the action data of the first action node based on the action waveform.

To more accurately determine the wearing tightness of the wearable device, that the wearable device determines feature data corresponding to the first action node based on the action data of each action node may be specifically implemented as follows:

S2032: The wearable device determines first feature data corresponding to the first action node based on the action data of the first action node.

The first feature data may include but is not limited to at least one of the following feature data: a wave peak quantity of an acceleration waveform or an angular velocity waveform, a wave trough quantity of the acceleration waveform or the angular velocity waveform, a peak value of the acceleration waveform or the angular velocity waveform, an acceleration average value or an angular velocity average value, a gradient of the acceleration waveform or the angular velocity waveform, and the like. It is clear that the first feature data may further include other feature data. This is not specifically limited in this embodiment of this application.

For example, S2032 may be specifically implemented as follows: The wearable device determines the action waveform formed by the action data of the first body part in the process in which the user performs the first action. The wearable device determines, based on the action waveform, a triggering moment of the first action node and first feature data corresponding to the triggering moment in first preset time.

The first preset time may be a period of time, and a length of the period of time is set based on an actual situation. This is not specifically limited in this embodiment of this application.

The foregoing example 1 is still used. In an example in which the first action is the jump action, correspondingly, the first action node of the first action may be a foot of the user leaving the ground or a foot of the user touching the ground. That the action data is an acceleration of the foot of the user, and the first action node is the foot of the user leaving the ground is used for description.

A: The wearable device determines an acceleration waveform formed by acceleration data of the foot in a process in which the user performs the jump action.

B: The wearable device determines, based on an acceleration waveform, that a moment at which the acceleration is greater than a preset acceleration is a first moment at which the foot leaves the ground, and determines acceleration data corresponding to the first moment in the first preset time. The wearable device calculates the first feature data in the first preset time based on the acceleration data corresponding to the first moment in the first preset time.

S2033: The wearable device determines the wearing tightness of the wearable device based on the first feature data corresponding to the first action node and the preset correspondence between the movement data and the wearing tightness.

To be specific, the wearable device determines the wearing tightness of the wearable device based on a correspondence between movement data (for example, standard data of the first action node) stored in the standard database and the wearing tightness and the first feature data corresponding to the first action node.

In the foregoing implementation, the standard data in the standard database may be pushed by a cloud server, downloaded by the wearable device, or pre-stored in the wearable device. The standard data may be the action data corresponding to the first action performed by an ordinary person, or may be historical data of the user of the wearable device. Specifically, the standard data may be filtered based on parameters such as a gender, a height, and a weight of the user. The standard data in the standard database may be specifically standard data of the first action node in a process in which the user performs the first action when the wearable device is in different wearing tightness. A type of the standard data may include but is not limited to at least one of the following data: the wave peak quantity of the acceleration waveform, the wave peak quantity of the angular velocity waveform, the wave trough quantity of the acceleration waveform, the wave trough quantity of the angular velocity waveform, the peak value of the acceleration waveform, the peak value of the angular velocity waveform, the acceleration average value, the angular velocity average value, the gradient of the acceleration waveform, the gradient of the angular velocity, and the like.

The foregoing example 1 is still used. In the example in which the first action is the jump action, correspondingly, the first action node of the first action may be the foot of the user leaving the ground or the foot of the user touching the ground. That the action data is the acceleration of the foot of the user, the first action node is the foot of the user leaving the ground, and the standard data is a wave peak quantity is used for description.

S2033 may be specifically implemented as follows: The wearable device determines the wearing tightness of the wearable device based on a corresponding wave peak quantity in the triggering moment in the first preset time and a pre-stored standard wave peak quantity of the action node that the foot leaves the ground in the process in which the user performs the jump action when the wearable device is in different wearing tightness.

In this embodiment of this application, the wearable device may determine the wearing tightness of the wearable device based on the action data of the user, and may detect the wearing tightness of the wearable device without an additional hardware apparatus driving an action of the wearable device. This can effectively avoid a problem of inaccurate collection of the action data, so that collection of the action data is more reliable, and a foundation for providing more professional guidance for the user is laid.

Manner 2: The wearable device determines, based on the action data of the first action and a wearing tightness model, the wearing tightness of the wearable device corresponding to the action data of the first action. The action data of the first action is used as an input of the wearing tightness model, the wearing tightness of the wearable device is used as an output of the wearing tightness model, and the wearing tightness model is obtained based on the movement data and the wearing tightness through training. A specific implementation of the model training is not specifically limited in this application.

Further, the method for detecting the wearing tightness of the wearable device provided in this embodiment of this application may further include: S204: The wearable device generates prompt information based on the wearing tightness, to notify the user of a detection result of the wearing tightness of the wearable device. The wearing tightness of the wearable device may be understood as a wearing tightness degree of the wearable device, or may be understood as a specific value. When the wearing tightness of the wearable device is understood as the wearing tightness degree of the wearable device, the wearing tightness of the wearable device may include that the wearable device is worn normally, that the wearable device is worn loosely, and that the wearable device is worn tightly. When the wearing tightness of the wearable device is understood as the specific value, S204 may be specifically implemented as follows: When the wearing tightness of the wearable device is greater than a threshold, the wearable device determines that the wearable device is worn tightly, and notifies the user of a detection result of worn tightly through first prompt information. When the wearing tightness of the wearable device is equal to or close to the threshold, the wearable device determines that the wearable device is worn normally, and notifies the user of a detection result of worn normally through second prompt information. When the wearing tightness of the wearable device is less than the threshold, the wearable device determines that the wearable device is worn loosely, and notifies the user of a wearing looseness detection result through third prompt information.

The first prompt information, the second prompt information, and the third prompt information may notify the user in different presentation forms. For example, the first prompt information, the second prompt information, and the third prompt information may be displayed to the user on a user interface (user interface, UI), or the user is notified through light of different colors emitted by an LED light, or the user is notified through different vibration intensities and/or vibration times, or the user is notified through a sound. It is clear that the user may be notified in a manner of combining different presentation forms. This is not specifically limited in this embodiment of this application.

For example, in a presentation form 1, a prompt may be provided on the user interface UI in a text and vibration presentation form. For example, FIG. 4 is a schematic diagram of an application scenario of a method for detecting wearing tightness of a wearable device according to an embodiment of this application. As shown in FIG. 4, when it is detected that the wearable device is worn normally, the wearable device displays generated second prompt information on a user interface (where for example, "worn normally" is displayed on the user interface UI), and the wearable device vibrates once, to notify the user that the wearable device is worn normally. When it is detected that the wearable device is worn loosely, the wearable device displays generated third prompt information on the user interface (where for example, "worn loosely" is displayed on the user interface UI), and the wearable device vibrates twice, to notify the user that the wearable device is worn loosely. It is clear that a quantity of vibration times of the wearable device may be set randomly. This is not specifically limited in this embodiment of this application.

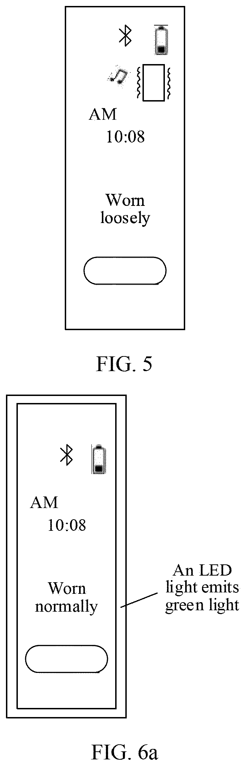

In a presentation form 2, the prompt may be provided on the user interface UI in a combination presentation form of text, vibration, and sound. For example, when it is detected that the wearable device is worn normally, the wearable device displays the generated second prompt information on the user interface (where for example, "worn normally" is displayed on the user interface UI), the wearable device vibrates once, and the wearable device sends out a first type of prompt tone at the same time to notify the user that the wearable device is worn normally. FIG. 5 is a schematic diagram of an application scenario of a method for detecting wearing tightness of a wearable device according to an embodiment of this application. As shown in FIG. 5, when it is detected that the wearable device is worn loosely, the wearable device displays generated third prompt information on a user interface (where for example, "worn loosely" is displayed on the user interface UI), the wearable device vibrates twice, and the wearable device sends out a second type of prompt tone at the same time, to notify the user that the wearable device is worn loosely. It is clear that a quantity of vibration times of the wearable device may be set randomly, and an alert tone may be randomly selected in different detection results. This is not specifically limited in this embodiment of this application.

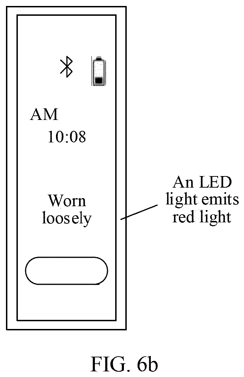

In a presentation form 3, the prompt may be provided on the user interface UI in a combination presentation form of a text and an LED light. For example, FIG. 6a is a schematic diagram of an application scenario of a method for detecting wearing tightness of a wearable device according to an embodiment of this application. As shown in FIG. 6a, when it is detected that the wearable device is worn normally, the wearable device displays generated second prompt information on a user interface (where for example, "worn normally" is displayed on the user interface UI), and an LED light disposed on the wearable device emits a first type of prompt light, to notify the user that the wearable device is worn normally. FIG. 6b is a schematic diagram of an application scenario of a method for detecting wearing tightness of a wearable device according to an embodiment of this application. As shown in FIG. 6b, when it is detected that the wearable device is worn loosely, the wearable device displays generated third prompt information on a user interface (where for example, "worn loose" is displayed on the user interface UI), and an LED light disposed on the wearable device emits a second type of prompt light, to notify the user that the wearable device is worn loosely. The first type of prompt light may be green light, and the second type of prompt light may be red light. It should be added herein that LED lights disposed on the wearable device may be arranged but are not limited to being arranged around a screen of the wearable device. In this way, when the user wears the wearable device in a dark place, the user wearing the wearable device can remind another user. This effectively prevents a risk of accidental collision.

It is clear that a presentation form of the sound may be specifically a presentation form of voice broadcast. In other words, a detection result of the wearing tightness of the wearable device may also be displayed in a voice broadcast form.

In this embodiment of this application, the detection result is notified to the user in different forms, so that a phenomenon that the wearable device of the user easily falls off and is lost in a movement process of the user can be effectively avoided, and a risk of collision or accidental collision of the user in the movement process can be avoided.

In some embodiments, the wearable device can evaluate movement energy of a user's movement based on movement data of the user. For example, in a running process of the user, the wearable device may evaluate the movement energy of user's running based on the movement data such as a running distance, a running speed, and running duration of the user. When the wearable device is worn loosely, the movement data, such as the running distance, the running speed, and the running duration of the user, collected by the wearable device is inaccurate, which affects accuracy of evaluating, by the wearable device, the movement energy of the user's movement based on the collected movement data of the user. To ensure that the wearable device outputs high accuracy of the movement energy of the user's movement in any state, as shown in FIG. 2, the method for detecting the wearing tightness of the wearable device according to this embodiment of this application further includes the following steps.

S205: The wearable device determines wearing tightness of the wearable device at each moment in first duration.

To be specific, in a process in which the user performs a movement for second preset time, the wearable device determines the wearing tightness of the wearable device at each moment in the second preset time (namely, the first duration).

S206: The wearable device determines second duration in which the wearable device is worn loosely based on the wearing tightness of the wearable device at each moment in the first duration.

The second duration may be continuous duration, or may be a sum of a plurality of discontinuous periods of duration.

In addition, the wearable device records, based on the wearing tightness of the wearable device at each moment in the second preset time, time when the wearable device is worn loosely, and sends out a reminder alarm.

This reminder alarm is used for reminding the user that the wearable device is worn loosely. The reminder alarm may include an alarm bell alarm, an alarm light alarm, or the like. It is clear that another form of reminder alarm may be included. This is not specifically limited in this embodiment of this application.

S207: The wearable device calculates second movement energy of the user in the first duration based on the first duration, the second duration, and first movement energy calculated by the wearable device.

The first movement energy is obtained through calculation when a wearing status of the wearable device in the first duration is normal or tight.

S207 may be specifically implemented as follows: The wearable device calculates, based on the time when the wearable device is worn loosely, the second duration in which the wearable device is worn loosely, and then determines a wearing looseness time proportion based on a ratio of the second duration in which the wearable device is worn loosely to the first duration. The wearable device determines the second movement energy of the user in the second preset time based on the wearing looseness time proportion and the first movement energy calculated by the wearable device.

For example, it is assumed that the movement of the user is running. In a running process of the user in the second preset time (namely, the foregoing first duration), the wearable device may determine the wearing tightness of the wearable device in real time. The wearable device evaluates the movement energy based on the movement data of the user collected in the second preset time. The wearable device calculates compensated movement energy based on the wearing looseness time proportion and the movement energy (namely, the foregoing first movement energy) evaluated by the wearable device in the running process of the user in the second preset time, where the compensated operating energy may be the wearing looseness time proportion multiplied by the evaluated movement energy. In this way, the wearable device may determine actual operating energy (namely, the foregoing second movement energy) of the user in the running process in the second preset time, where the actual operating energy may be a difference between the evaluated operating energy and the compensated operating energy.

Therefore, in this embodiment of this application, the wearable device may evaluate the movement energy of the user in the second preset time. When the wearable device is worn loosely, the wearable device may determine the actual movement energy of the movement of the user in the second preset time based on the wearing looseness time proportion and the evaluated movement energy, so that the movement data of the user is more reliable, to remind the user to pay attention to adjusting the wearing tightness of the wearable device during next running.

The following describes in detail, with reference to an application scenario, the method for detecting the wearing tightness of the wearable device provided in this embodiment of this application. A running application scenario is used as an example:

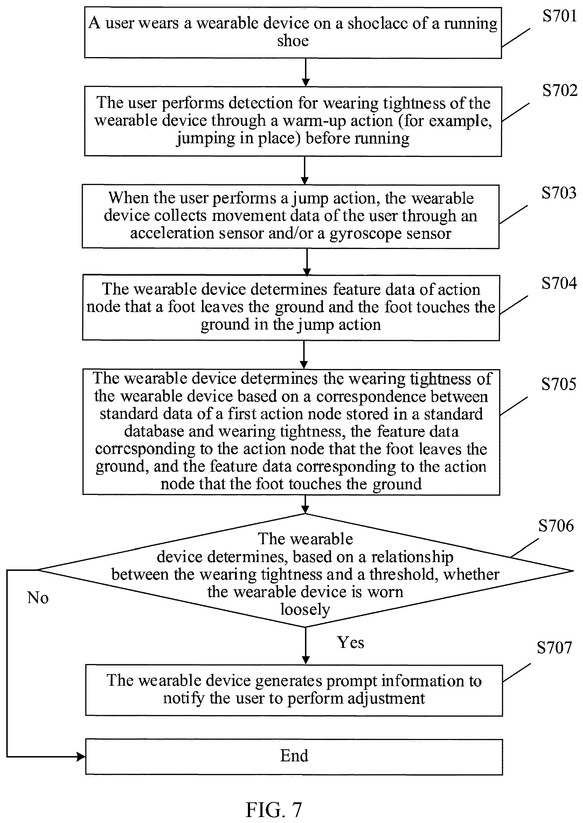

FIG. 7 is a schematic flowchart of a method for detecting wearing tightness of a wearable device in an application scenario according to an embodiment of this application before running. As shown in FIG. 7, the method includes the following steps. S701: A user wears the wearable device on a shoelace of a running shoe. S702: The user performs detection for the wearing tightness of the wearable device through a warm-up action (for example, jumping in place) before the running. S703: When the user performs a jump action, the wearable device collects movement data of the user through an acceleration sensor and/or a gyroscope sensor. S704: The wearable device identifies action data of the jump action from the collected movement data, and determines feature data of actions nodes that a foot leaves the ground and the foot touches the ground in the jump action. The foregoing first action is the jump action, and the foregoing action nodes include: the foot leaves the ground and the foot touches the ground. S704 may be specifically: The wearable device determines an acceleration waveform formed by acceleration data of the foot in a process in which the user performs the jump action. The wearable device determines, based on the acceleration waveform, that a moment at which an acceleration is greater than a preset acceleration is a first moment at which the foot leaves the ground, and determines acceleration data corresponding to the first moment in first preset time. The wearable device calculates feature data in the first preset time based on the acceleration data corresponding to the first moment in the first preset time. The feature data may include but is not limited to at least one of the following feature data: a wave peak quantity, a wave trough quantity, a peak value, an acceleration average value, a gradient of an acceleration waveform, and the like. S705: The wearable device determines the wearing tightness of the wearable device based on a correspondence between standard data of a first action node (for example, the foot leaves the ground and the foot touches the ground) stored in a standard database and wearing tightness, the feature data corresponding to the action node that foot leaves the ground, and the feature data corresponding to the action node that the foot touches the ground. S706: The wearable device determines, based on a relationship between the wearing tightness and a threshold, whether the wearable device is worn loosely. When the wearing tightness of the wearable device is less than the threshold, the wearable device determines that the wearable device is worn loosely; when the wearing tightness of the wearable device is equal to or close to the threshold, the wearable device determines that the wearable device is worn normally; or when the wearing tightness of the wearable device is greater than the threshold, the wearable device determines that the wearable device is worn tightly. If the wearable device determines that the wearable device is not worn loosely, the wearable device performs an end procedure; or if the wearable device determines that the wearable device is worn loosely, the wearable device performs S707. S707: The wearable device generates prompt information to notify the user to perform adjustment.

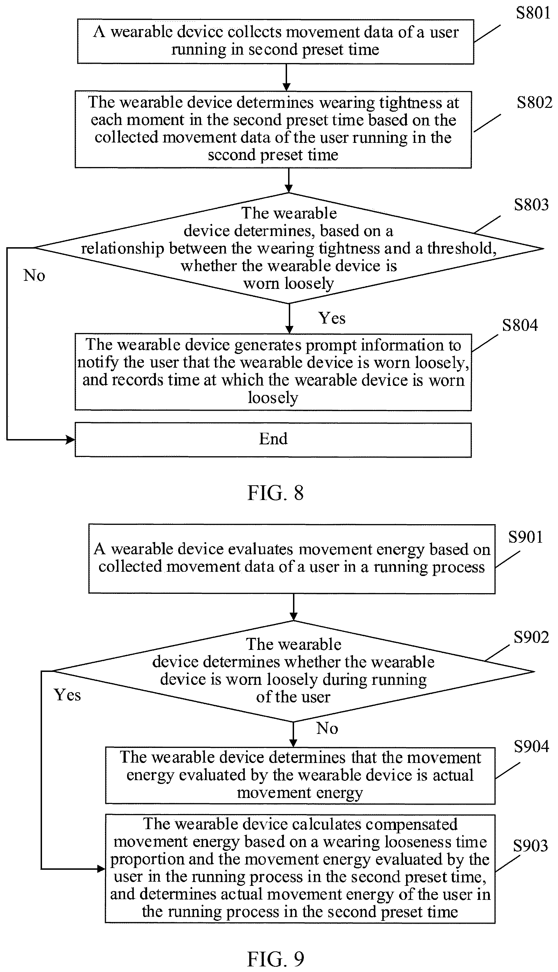

FIG. 8 is a schematic flowchart of a method for detecting wearing tightness of a wearable device in an application scenario according to an embodiment of this application in the running process. As shown in FIG. 8, the method includes the following steps. S801: The wearable device collects the movement data of the user running in second preset time (namely, the foregoing first duration). S802: The wearable device determines the wearing tightness at each moment in the second preset time based on the collected movement data of the user running in the second preset time. S803: The wearable device determines, based on a relationship between the wearing tightness and a threshold, whether the wearable device is worn loosely. If the wearable device determines that the wearable device is not worn loosely, the wearable device performs an end procedure; or if the wearable device determines that the wearable device is worn loosely, the wearable device performs S804. S804: The wearable device generates prompt information to notify the user that the wearable device is worn loosely, and records time at which the wearable device is worn loosely.

FIG. 9 is a schematic flowchart of a method for detecting wearing tightness of a wearable device in an application scenario according to an embodiment of this application after running is completed. As shown in FIG. 9, the method includes the following steps. S901: The wearable device evaluates movement energy based on the collected movement data of the user in the running process. S902: The wearable device determines whether the wearable device is worn loosely during running of the user. If the wearable device determines that the wearable device is worn loosely during the running of the user, S903 is performed; or if the wearable device determines that the wearable device is not worn loosely during the running of the user, S904 is performed. S903: The wearable device determines wearing looseness time in the second preset time, and determines a wearing looseness time proportion based on a ratio of the wearing looseness time to the second preset time. The wearable device calculates compensated movement energy based on the wearing looseness time proportion and the movement energy (namely, the foregoing first movement energy) evaluated by the wearable device in the running process of the user in the second preset time, where the compensated operating energy may be the wearing looseness time proportion multiplied by the evaluated movement energy. The wearable device determines actual operating energy (namely, the foregoing second movement energy) of the user in the running process in the second preset time, where the actual operating energy may be a difference between the evaluated operating energy and the compensated operating energy. S904: The wearable device determines that the movement energy evaluated by the wearable device is the actual movement energy.

In a second case, when there are at least two execution bodies of the method for detecting the wearing tightness of the wearable device, the execution bodies of the method for detecting the wearing tightness of the wearable device may include a wearable device and a non-wearable device.

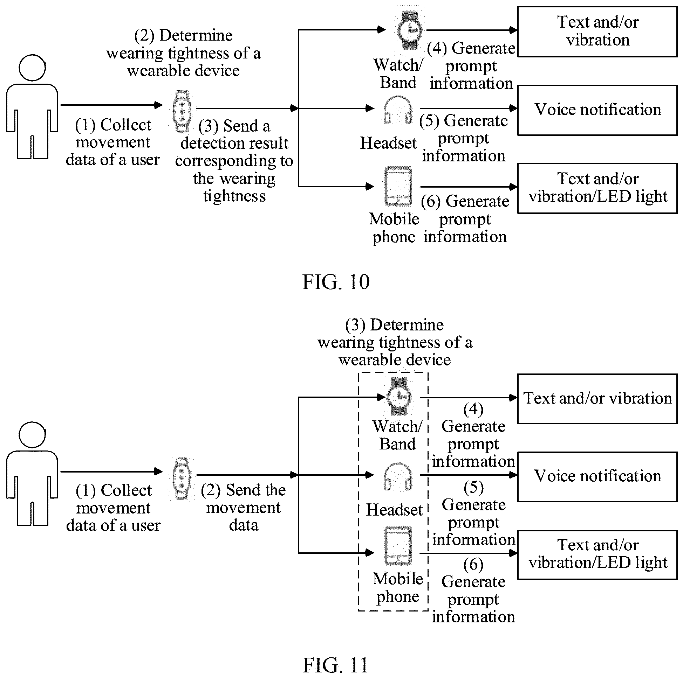

A cooperative operation between the wearable device and the non-wearable device may be implemented in a plurality of manners. Details may be as follows:
Manner 1: FIG. 10 is a schematic flowchart of a method for detecting wearing tightness of a wearable device according to an embodiment of this application. As shown in FIG. 10, the method may include the following steps. (1) When detecting that the wearable device is in a worn state, the wearable device collects movement data of a user. (2) The wearable device identifies action data of a first action from the collected movement data, and determines first feature data corresponding to a first action node of the first action of the user. Specifically, the wearable device identifies the action data of the first action from collected action data of the user, and determines an action waveform formed by action data of a first body part in a process in which the user performs the first action. The wearable device determines, based on the action waveform, a triggering moment of the first action node and first feature data corresponding to the triggering moment in first preset time. The wearable device determines the wearing tightness of the wearable device based on a correspondence between standard data of the first action node stored in a standard database and the wearing tightness and the first feature data corresponding to the first action node. (3) The wearable device sends a detection result corresponding to the wearing tightness to the non-wearable device. Specifically, the wearable device may send the detection result corresponding to the wearing tightness to the non-wearable device through Bluetooth. It is clear that another transmission manner may also be used. This is not specifically limited in this embodiment of this application.

For different specific types of non-wearable devices, the non-wearable device receives the detection result that is of the wearing tightness of the wearable device and that is sent by the wearable device, and notifies the user of the detection result in different manners. Specifically:
(4) For example, if the non-wearable device is a watch or a band, the watch or the band receives the detection result that is of the wearing tightness of the wearable device and that is sent by the wearable device, and generates prompt information based on the detection result. The prompt information may be presented on a user interface UI through a text, and/or may be notified in a vibration presentation form.

For example, as shown in FIG. 4, when it is detected that the wearable device is worn normally, the watch or the band displays the generated prompt information on the user interface (where for example, "worn normally" is displayed on the user interface UI), and the watch or the band vibrates once, to notify the user that the wearable device is worn normally. When it is detected that the wearable device is worn loosely, the watch or the band displays the generated prompt information on the user interface (where for example, "worn loosely" is displayed on the user interface UI), and the wearable device vibrates twice, to notify the user that the wearable device is worn loosely. It is clear that a quantity of vibration times of the wearable device may be set randomly. This is not specifically limited in this embodiment of this application.

(5) If the non-wearable device is a headset, the headset receives the detection result that is of the wearing tightness of the wearable device and that is sent by the wearable device, and generates prompt information based on the detection result. The prompt information may be notified in a voice presentation form.

(6) If the non-wearable device is a mobile phone, the mobile phone receives the detection result that is of the wearing tightness of the wearable device and that is sent by the wearable device, and generates prompt information based on the detection result. The prompt information may be presented on the user interface UI through a text, and/or may be notified in a vibration/LED light presentation form.

For example, as shown in FIG. 6a, when it is detected that the wearable device is worn normally, the mobile phone displays the generated prompt information on the user interface (where for example, "worn normally" is displayed on the user interface UI), and an LED light disposed on the mobile phone emits a first type of prompt light, to notify the user that the wearable device is worn normally. As shown in FIG. 6b, when it is detected that the wearable device is worn loosely, the mobile phone displays the generated prompt information on the user interface (where for example, "worn loosely" is displayed on the user interface UI), and the LED light disposed on the mobile phone emits a second type of prompt light, to notify the user that the wearable device is worn loosely. The first type of prompt light may be green light, and the second type of prompt light may be red light. It should be added herein that, it is clear that a prompt manner of the mobile phone may also be a screen-on prompt of the mobile phone.

It is clear that a presentation form of a sound may be specifically a presentation form of voice broadcast. In other words, the mobile phone may display the detection result of the wearing tightness of the wearable device in a voice broadcast form.

For specific implementation of each step, refer to related content in the foregoing embodiments. Details are not described again in this embodiment of this application.

### Manner 2:

FIG. 11 is a schematic flowchart of a method for detecting wearing tightness of a wearable device according to an embodiment of this application. As shown in FIG. 11, the method may include the following steps. (1) When detecting that the wearable device is in a worn state, the wearable device collects movement data of a user. (2) The wearable device sends the movement data to a non-wearable device. Specifically, the wearable device may send the movement data to the non-wearable device through Bluetooth. It is clear that another transmission manner may also be used. This is not specifically limited in this embodiment of this application. (3) The non-wearable device receives the movement data of the user sent by the wearable device, identifies action data of a first action from the movement data, and determines first feature data corresponding to a first action node of the first action of the user. Specifically, the non-wearable device determines, based on the action data of the user, an action waveform formed by action data of a first body part in a process in which the user performs the first action. The non-wearable device determines, based on the action waveform, a triggering moment of the first action node and first feature data corresponding to the triggering moment in first preset time. The non-wearable device determines the wearing tightness of the wearable device based on a correspondence between standard data of the first action node stored in a standard database and the wearing tightness and the first feature data corresponding to the first action node.

For different specific types of non-wearable devices, the non-wearable device notifies the user of a detection result of the wearing tightness of the wearable device in different manners. Specifically:
(4) For example, if the non-wearable device is a watch or a band, the watch or the band generates prompt information based on the wearing tightness of the wearable device. The prompt information may be presented on a user interface UI through a text, and/or may be notified in a vibration presentation form.

For example, as shown in FIG. 4, when it is detected that the wearable device is worn normally, the watch or the band displays the generated prompt information on the user interface (where for example, "worn normally" is displayed on the user interface UI), and the watch or the band vibrates once, to notify the user that the wearable device is worn normally. When it is detected that the wearable device is worn loosely, the watch or the band displays the generated prompt information on the user interface (where for example, "worn loosely" is displayed on the user interface UI), and the wearable device vibrates twice, to notify the user that the wearable device is worn loosely. It is clear that a quantity of vibration times of the wearable device may be set randomly. This is not specifically limited in this embodiment of this application.

(5) If the non-wearable device is a headset, the headset generates prompt information based on the wearing tightness of the wearable device, and the prompt information may be notified in a voice presentation form.

(6) If the non-wearable device is a mobile phone, the mobile phone generates prompt information based on the wearing tightness of the wearable device. The prompt information may be presented on the user interface UI through a text, and/or may be notified in a vibration/LED light presentation form.

For example, as shown in FIG. 6a, when it is detected that the wearable device is worn normally, the mobile phone displays the generated prompt information on the user interface (where for example, "worn normally" is displayed on the user interface UI), and an LED light disposed on the mobile phone emits a first type of prompt light, to notify the user that the wearable device is worn normally. As shown in FIG. 6b, when it is detected that the wearable device is worn loosely, the mobile phone displays the generated prompt information on the user interface (where for example, "worn loosely" is displayed on the user interface UI), and the LED light disposed on the mobile phone emits a second type of prompt light, to notify the user that the wearable device is worn loosely. The first type of prompt light may be green light, and the second type of prompt light may be red light. It should be added herein that, it is clear that a prompt manner of the mobile phone may also be a screen-on prompt of the mobile phone.

It is clear that a presentation form of a sound may be specifically a presentation form of voice broadcast. In other words, the mobile phone may display the detection result of the wearing tightness of the wearable device in a voice broadcast form.

For specific implementation of each step, refer to related content in the foregoing embodiments. Details are not described again in this embodiment of this application.

In this embodiment of this application, a function of detecting the wearing tightness of the wearable device and a prompt function are extended to a plurality of devices. Through a cooperative operation between the plurality of devices, a problem of the wearing tightness of the user is resolved, and experience of linkage between a plurality of devices of the user is improved.

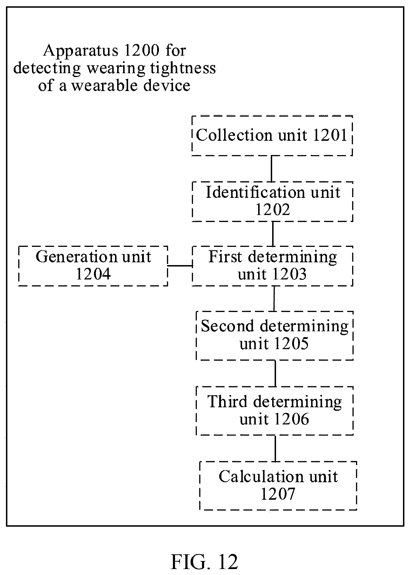

FIG. 12 shows an apparatus for detecting wearing tightness of a wearable device according to an embodiment of this application. The apparatus 1200 may include:
a collection unit 1201, configured to collect movement data of a user when it is detected that the wearable device is in a worn state;
an identification unit 1202, configured to identify action data of a first action in the movement data of the user; and
a first determining unit 1203, configured to determine the wearing tightness of the wearable device corresponding to the action data of the first action.

In a possible implementation, the first determining unit 1203 is further configured to:
determine the wearing tightness of the wearable device based on the action data of the first action and a preset correspondence between the movement data and the wearing tightness.

In a possible implementation, the first determining unit 1203 is further configured to:
identify action data of a first action node in the action data of the first action;
determine first feature data corresponding to the first action node based on the action data of the first action node; and
determine the wearing tightness of the wearable device based on the first feature data and the preset correspondence between the movement data and the wearing tightness.

In an implementation, the first feature data includes at least one of a wave peak quantity of an acceleration waveform, a wave peak quantity of an angular velocity waveform, a wave trough quantity of the acceleration waveform, a wave trough quantity of the angular velocity waveform, a peak value of the acceleration waveform, a peak value of the angular velocity waveform, an acceleration average value, an angular velocity average value, a gradient of the acceleration waveform, and a gradient of the angular velocity waveform.

In a possible implementation, the first determining unit 1203 is further configured to:
determine, based on the action data of the first action and a wearing tightness model, the wearing tightness of the wearable device corresponding to the action data of the first action.

The action data of the first action is used as an input of the wearing tightness model, and the wearing tightness of the wearable device is used as an output of the wearing tightness model.

In some embodiments, the apparatus for detecting the wearing tightness of the wearable device provided in this embodiment of this application may further include:
a generation unit 1204, configured to generate prompt information based on the wearing tightness of the wearable device, where the prompt information presents a detection result of the wearing tightness of the wearable device in a preset presentation form.

The preset presentation form includes one or a combination of the following: a sound presentation form, a text presentation form, a vibration presentation form, and a light presentation form.

In a possible implementation, the wearing tightness of the wearable device includes: the wearable device is worn normally; the wearable device is worn loosely; and the wearable device is worn tightly.

In some embodiments, the apparatus for detecting the wearing tightness of the wearable device provided in this embodiment of this application may further include:
a second determining unit 1205, configured to determine wearing tightness of the wearable device at each moment in first duration;
a third determining unit 1206, configured to determine second duration in which the wearable device is worn loosely based on the wearing tightness of the wearable device at each moment in the first duration; and
a calculation unit 1207, configured to calculate second movement energy of the user in the first duration based on the first duration, the second duration, and first movement energy calculated by the wearable device, where the first movement energy is obtained through calculation when a wearing status of the wearable device in the first duration is normal or tight.

In an implementation, the first action includes a jump action, and the first action node includes: a foot leaves the ground and/or the foot touches the ground; or
the first action includes an arm swinging action, and the first action node includes: an arm swings on a front side of a body at a specified position and/or the arm swings on a rear side of the body at a specified position.

In a possible implementation, the wearable device is configured to be worn on the foot or a shoe of the user.

Optionally, the foregoing apparatus 1200 may be implemented through code or through a circuit. Specifically, the apparatus may be an entire system of a terminal device. For example, the collection unit 1201 may be a collection circuit, or may be implemented by a sensor (the acceleration sensor 150A and/or the gyroscope sensor 150B shown in FIG. 1). The identification unit 1202, the first determining unit 1203, the generation unit 1204, the second determining unit 1205, the third determining unit 1206, and the calculation unit 1207 may be processors (the processor 110 shown in FIG. 1).

Specifically, in the possible design, all related content of the steps related to an electronic device in the method embodiments shown in FIG. 1 to FIG. 11 may be referenced to function descriptions of corresponding functional modules. Details are not described herein again. The electronic device in this possible design is configured to: perform functions of an electronic device in the methods for detecting wearing tightness of a wearable device shown in FIG. 1 to FIG. 11, and therefore, can achieve a same effect as the foregoing method for detecting the wearing tightness of the wearable device.

An embodiment of this application further provides a chip system. The chip system includes at least one processor and at least one interface circuit. The processor and the interface circuit may be interconnected through a line. For example, the interface circuit may be configured to receive a signal from another apparatus (for example, a memory). For another example, the interface circuit may be configured to send a signal to another apparatus (for example, the processor). For example, the interface circuit may read instructions stored in the memory, and send the instructions to the processor. When the instructions are executed by the processor, an electronic device may be enabled to perform the steps performed by the wearable device in the foregoing embodiments. It is clear that the chip system may further include another discrete device. This is not specifically limited in this embodiment of this application.

An embodiment of this application further provides an apparatus. The apparatus is included in an electronic device, and the apparatus has a function of implementing behavior of the electronic device in any method in the foregoing embodiments. The function may be implemented by hardware, or may be implemented by hardware executing corresponding software. The hardware or the software includes at least one module or unit corresponding to the foregoing function, for example, a detection module or unit, and a determining module or unit.

An embodiment of this application further provides a computer-readable storage medium, including computer instructions. When the computer instructions are run on an electronic device, the electronic device is enabled to perform any method in the foregoing embodiments.

An embodiment of this application further provides a computer program product. When the computer program product is run on a computer, the computer is enabled to perform any method in the foregoing embodiments.

It may be understood that to implement the foregoing functions, the terminal or the like includes corresponding hardware structures and/or software modules for performing the functions. A person skilled in the art should be easily aware that, in combination with the examples described in embodiments disclosed in this specification, units, algorithms, and steps may be implemented by hardware or a combination of hardware and computer software in embodiments of this application. Whether a function is performed by the hardware or the hardware driven by the computer software depends on particular applications and design constraints of the technical solutions. A person skilled in the art may use different methods to implement the described functions for each particular application, but it should not be considered that the implementation goes beyond the scope of the embodiments of the present invention.

In embodiments of this application, the terminal or the like may be divided into function modules based on the foregoing method examples. For example, each function module may be obtained through division based on each corresponding function, or two or more functions may be integrated into one processing module. The foregoing integrated module may be implemented in a form of hardware, or may be implemented in a form of a software functional module. It should be noted that in embodiments of the present invention, module division is an example, and is merely a logical function division. During actual implementation, another division manner may be used.

The foregoing descriptions about implementations allow a person skilled in the art to clearly understand that, for the purpose of convenient and brief description, division of the foregoing function modules is taken as an example for illustration. In actual application, the foregoing functions can be allocated to different modules and implemented according to a requirement, in other words, an inner structure of an apparatus is divided into different function modules to implement all or some of the functions described above. For a detailed working process of the foregoing system, apparatus, and unit, refer to a corresponding process in the foregoing method embodiments, and details are not described herein again.

In embodiments of this application, functional units in embodiments of this application may be integrated into one processing unit, or each of the units may exist alone physically, or two or more units are integrated into one unit. The integrated unit may be implemented in a form of hardware, or may be implemented in a form of a software functional unit.

When the integrated unit is implemented in the form of the software functional unit and sold or used as an independent product, the integrated unit may be stored in a computer-readable storage medium. Based on such an understanding, the technical solutions of embodiments of this application essentially, or the part contributing to the current technology, or all or some of the technical solutions may be implemented in a form of a software product. The computer software product is stored in a storage medium and includes several instructions to instruct a computer device (which may be a personal computer, a server, a network device, or the like) or a processor to perform all or some of the steps of the methods described in embodiments of this application. The foregoing storage medium includes any medium that can store program code, such as a flash memory, a removable hard disk, a read-only memory, a random access memory, a magnetic disk, or an optical disc.

The foregoing descriptions are merely specific implementations of this application, but are not intended to limit the protection scope of this application. Any variation or replacement within the technical scope disclosed in this application shall fall within the protection scope of this application. Therefore, the protection scope of this application shall be subject to the protection scope of the claims.

## Claims

1. A method for detecting wearing tightness of a wearable device, wherein the method comprises:
collecting (1), by the wearable device, movement data of a user when it is detected that the wearable device is in a worn state, wherein the movement data of the user comprises an acceleration and/or an angular velocity;
identifying (2), by the wearable device, action data of a first action in the movement data of the user, wherein the first action is an action of the user in a movement process, the first action corresponds to a position where the wearable device is worn, and when the wearable device is worn on an arm or a wrist of the user, the first action comprises an arm swinging action, and when the wearable device is worn on an ankle or foot or a shoe of the user, the first action comprises a jump action; and
identifying (2), by the wearable device, action data of a first action node in the action data of the first action, wherein when the first action comprises an arm swinging action the first action node includes: an arm swings on a front side of a body at a specified position and/or the arm swings on a rear side of the body at a specified position, and when the first action comprises a jump action the first action node includes: a foot leaves the ground and/or the foot touches the ground;
determining (2), by the wearable device, first feature data corresponding to the first action node based on the action data of the first action node, wherein the first feature data comprises at least one of a wave peak quantity of an acceleration waveform, a wave peak quantity of an angular velocity waveform, a wave trough quantity of the acceleration waveform, a wave trough quantity of the angular velocity waveform, a peak value of the acceleration waveform, a peak value of the angular velocity waveform, an acceleration average value, an angular velocity average value, a gradient of the acceleration waveform, and a gradient of the angular velocity waveform;
determining (2), by the wearable device, the wearing tightness of the wearable device corresponding to the action data of the first action, comprising determining the wearing tightness of the wearable device based on a correspondence between preset data of the first action node and the wearing tightness and the first feature data corresponding to the first action node;
sending (3), by the wearable device, a detection result of the wearing tightness of the wearable device to a non-wearable device;
receiving (4), by the non-wearable device, the detection result of the wearing tightness of the wearable device; and
generating (4), by the non-wearable device, prompt information based on the detection result of the wearing tightness of the wearable device for notifying the user of the detection result of the wearing tightness of the wearable device.

2. The method according to claim 1, wherein the determining the wearing tightness of the wearable device corresponding to the action data of the first action comprises:
determining, based on the action data of the first action and a wearing tightness model, the wearing tightness of the wearable device corresponding to the action data of the first action, wherein
the action data of the first action is used as an input of the wearing tightness model, and the wearing tightness of the wearable device is used as an output of the wearing tightness model.

3. The method according to any one of claims 1 to 2, wherein the prompt information presents a detection result of the wearing tightness of the wearable device in a preset presentation form, wherein
the preset presentation form comprises one or a combination of the following: a sound presentation form, a text presentation form, a vibration presentation form, and a light presentation form.

4. The method according to any one of claims 1 to 3, wherein the wearing tightness of the wearable device comprises: the wearable device is worn normally; the wearable device is worn loosely; and the wearable device is worn tightly.

5. The method according to any one of claims 1 to 4, further comprising:
determining wearing tightness of the wearable device at each moment in first duration;
determining second duration in which the wearable device is worn loosely based on the wearing tightness of the wearable device at each moment in the first duration; and
calculating second movement energy of the user in the first duration based on the first duration, the second duration, and first movement energy calculated by the wearable device, wherein the first movement energy is obtained through calculation when a wearing status of the wearable device in the first duration is normal or tight.

6. A system for detecting wearing tightness of a wearable device, wherein the system comprises:
the wearable device, configured to:
collect (1) movement data of a user when it is detected that the wearable device is in a worn state, wherein the movement data of the user may comprises an acceleration and/or an angular velocity;
identify (2) action data of a first action in the movement data of the user, wherein the first action is an action of the user in a movement process, the first action corresponds to a position where the wearable device is worn, and when the wearable device is worn on an arm or a wrist of the user, the first action comprises an arm swinging action, and when the wearable device is worn on an ankle or a shoe of the user, the first action comprises a jump action;
identify (2) action data of a first action node in the action data of the first action, wherein when the first action comprises an arm swinging action the first action node includes: an arm swings on a front side of a body at a specified position and/or the arm swings on a rear side of the body at a specified position, and when the first action comprises a jump action the first action node includes: a foot leaves the ground and/or the foot touches the ground;
determine (2) first feature data corresponding to the first action node based on the action data of the first action node, wherein the first feature data comprises at least one of a wave peak quantity of an acceleration waveform, a wave peak quantity of an angular velocity waveform, a wave trough quantity of the acceleration waveform, a wave trough quantity of the angular velocity waveform, a peak value of the acceleration waveform, a peak value of the angular velocity waveform, an acceleration average value, an angular velocity average value, a gradient of the acceleration waveform, and a gradient of the angular velocity waveform
determine (2) the wearing tightness of the wearable device corresponding to the action data of the first action, comprising a determination of the wearing tightness of the wearable device based on a correspondence between preset data of the first action node and the wearing tightness and the first feature data corresponding to the first action node; and
send (3) a detection result of the wearing tightness of the wearable device to a non-wearable device; and
the non-wearable device, configured to:
receive (4) the detection result of the wearing tightness of the wearable device, and
generate (4) prompt information based on the detection result of the wearing tightness of the wearable device, wherein the prompt information is for notifying the user of the detection result of the wearing tightness of the wearable device.

7. A computer-readable storage medium, wherein the computer-readable storage medium comprises computer instructions, and when the computer instructions are run on a system according to claim 6, the system is enabled to perform the method for detecting wearing tightness of a wearable device according to any one of claims 1 to 5.

## Patentansprüche

1. Verfahren zum Detektieren einer Tragefestigkeit einer tragbaren Vorrichtung, wobei das Verfahren umfasst:
Sammeln (1) von Bewegungsdaten eines Benutzers durch die tragbare Vorrichtung, wenn detektiert wird, dass sich die tragbare Vorrichtung in einem getragenen Zustand befindet, wobei die Bewegungsdaten des Benutzers eine Beschleunigung und/oder eine Winkelgeschwindigkeit umfassen;
Identifizieren (2) von Aktionsdaten einer ersten Aktion in den Bewegungsdaten des Benutzers durch die tragbare Vorrichtung, wobei die erste Aktion eine Aktion des Benutzers in einem Bewegungsprozess ist, die erste Aktion einer Position, an der die tragbare Vorrichtung getragen wird, entspricht, und, wenn die tragbare Vorrichtung an einem Arm oder einem Handgelenk des Benutzers getragen wird, die erste Aktion eine Armschwingaktion umfasst, und, wenn die tragbare Vorrichtung an einem Knöchel oder Fuß oder einem Schuh des Benutzers getragen wird, die erste Aktion eine Sprungaktion umfasst; und
Identifizieren (2) von Aktionsdaten eines ersten Aktionsknotens in den Aktionsdaten der ersten Aktion durch die tragbare Vorrichtung, wobei, wenn die erste Aktion eine Armschwingaktion umfasst, der erste Aktionsknoten einschließt: ein Arm schwingt an einer Vorderseite eines Körpers an einer spezifizierten Position und/oder der Arm schwingt an einer Rückseite des Körpers an einer spezifizierten Position, und, wenn die erste Aktion eine Sprungaktion umfasst, der erste Aktionsknoten einschließt: ein Fuß verlässt den Boden und/oder der Fuß berührt den Boden;
Bestimmen (2) von ersten dem ersten Aktionsknoten entsprechenden Merkmalsdaten durch die tragbare Vorrichtung basierend auf den Aktionsdaten des ersten Aktionsknotens, wobei die ersten Merkmalsdaten mindestens eines von einer Wellenberganzahl einer Beschleunigungswellenform, einer Wellenberganzahl einer Winkelgeschwindigkeitswellenform, einer Wellentalanzahl der Beschleunigungswellenform, einer Wellentalanzahl der Winkelgeschwindigkeitswellenform, einem Gipfelwert der Beschleunigungswellenform, einem Gipfelwert der Winkelgeschwindigkeitswellenform, einem Beschleunigungsdurchschnittswert, einem Winkelgeschwindigkeitsdurchschnittswert, einem Gradienten der Beschleunigungswellenform und einem Gradienten der Winkelgeschwindigkeitswellenform umfassen;
Bestimmen (2) der den Aktionsdaten der ersten Aktion entsprechenden Tragefestigkeit der tragbaren Vorrichtung durch die tragbare Vorrichtung, das das Bestimmen der Tragefestigkeit der tragbaren Vorrichtung basierend auf einer Entsprechung zwischen voreingestellten Daten des ersten Aktionsknotens und der Tragefestigkeit und den ersten dem ersten Aktionsknoten entsprechenden Merkmalsdaten umfasst;
Senden (3) eines Detektionsergebnisses der Tragefestigkeit der tragbaren Vorrichtung an eine nicht tragbare Vorrichtung durch die tragbare Vorrichtung;
Empfangen (4) des Detektionsergebnisses der Tragefestigkeit der tragbaren Vorrichtung durch die nicht tragbare Vorrichtung; und
Erzeugen (4) von Aufforderungsinformationen durch die nicht tragbare Vorrichtung basierend auf dem Detektionsergebnis der Tragefestigkeit der tragbaren Vorrichtung zum Benachrichtigen des Benutzers über das Detektionsergebnis der Tragefestigkeit der tragbaren Vorrichtung.

2. Verfahren nach Anspruch 1, wobei das Bestimmen der Tragefestigkeit der tragbaren Vorrichtung entsprechend den Aktionsdaten der ersten Aktion umfasst:
Bestimmen der Tragefestigkeit der tragbaren Vorrichtung entsprechend den Aktionsdaten der ersten Aktion basierend auf den Aktionsdaten der ersten Aktion und einem Tragefestigkeitsmodell, wobei
die Aktionsdaten der ersten Aktion als eine Eingabe des Tragefestigkeitsmodells verwendet werden, und die Tragefestigkeit der tragbaren Vorrichtung als eine Ausgabe des Tragefestigkeitsmodells verwendet wird.

3. Verfahren nach einem der Ansprüche 1 bis 2, wobei die Aufforderungsinformation ein Detektionsergebnis der Tragefestigkeit der tragbaren Vorrichtung in einer voreingestellten Präsentationsform präsentiert, wobei
die voreingestellte Präsentationsform eine oder eine Kombination der Folgenden umfasst: eine Geräuschpräsentationsform, eine Textpräsentationsform, eine Vibrationspräsentationsform und eine Lichtpräsentationsform.

4. Verfahren nach einem der Ansprüche 1 bis 3, wobei die Tragefestigkeit der tragbaren Vorrichtung umfasst: die tragbare Vorrichtung wird normal getragen; die tragbare Vorrichtung wird locker getragen; und die tragbare Vorrichtung wird fest getragen.

5. Verfahren nach einem der Ansprüche 1 bis 4, das ferner umfasst:
Bestimmen der Tragefestigkeit der tragbaren Vorrichtung zu jedem Zeitpunkt in einer ersten Zeitdauer;
Bestimmen einer zweiten Zeitdauer, in der die tragbare Vorrichtung locker getragen wird, basierend auf der Tragefestigkeit der tragbaren Vorrichtung zu jedem Zeitpunkt in der ersten Zeitdauer; und
Berechnen einer zweiten Bewegungsenergie des Benutzers in der ersten Zeitdauer basierend auf der ersten Zeitdauer, der zweiten Zeitdauer und einer ersten Bewegungsenergie, die durch die tragbare Vorrichtung berechnet wird, wobei die erste Bewegungsenergie durch eine Berechnung, wenn ein Tragestatus der tragbaren Vorrichtung in der ersten Zeitdauer normal oder fest ist, erhalten wird.

6. System zum Detektieren der Tragefestigkeit einer tragbaren Vorrichtung, wobei das System umfasst:
die tragbare Vorrichtung, konfiguriert zum:
Sammeln (1) von Bewegungsdaten eines Benutzers, wenn detektiert wird, dass sich die tragbare Vorrichtung in einem getragenen Zustand befindet, wobei die Bewegungsdaten des Benutzers eine Beschleunigung und/oder eine Winkelgeschwindigkeit umfassen können;
Identifizieren (2) von Aktionsdaten einer ersten Aktion in den Bewegungsdaten des Benutzers, wobei die erste Aktion eine Aktion des Benutzers in einem Bewegungsprozess ist, die erste Aktion einer Position, an der die tragbare Vorrichtung getragen wird, entspricht, und, wenn die tragbare Vorrichtung an einem Arm oder einem Handgelenk des Benutzers getragen wird, die erste Aktion eine Armschwingaktion umfasst, und, wenn die tragbare Vorrichtung an einem Knöchel oder einem Schuh des Benutzers getragen wird, die erste Aktion eine Sprungaktion umfasst;
Identifizieren (2) von Aktionsdaten eines ersten Aktionsknotens in den Aktionsdaten der ersten Aktion, wobei, wenn die erste Aktion eine Armschwingaktion umfasst, der erste Aktionsknoten einschließt: ein Arm schwingt an einer Vorderseite eines Körpers an einer spezifizierten Position und/oder der Arm schwingt an einer Rückseite des Körpers an einer spezifizierten Position, und, wenn die erste Aktion eine Sprungaktion umfasst, der erste Aktionsknoten einschließt: ein Fuß verlässt den Boden und/oder der Fuß berührt den Boden;
Bestimmen (2) von ersten dem ersten Aktionsknoten entsprechenden Merkmalsdaten basierend auf den Aktionsdaten des ersten Aktionsknotens, wobei die ersten Merkmalsdaten mindestens eines von einer Wellenberganzahl einer Beschleunigungswellenform, einer Wellenberganzahl einer Winkelgeschwindigkeitswellenform, einer Wellentalanzahl der Beschleunigungswellenform, einer Wellentalanzahl der Winkelgeschwindigkeitswellenform, einem Gipfelwert der Beschleunigungswellenform, einem Gipfelwert der Winkelgeschwindigkeitswellenform, einem Beschleunigungsdurchschnittswert, einem Winkelgeschwindigkeitsdurchschnittswert, einem Gradienten der Beschleunigungswellenform und einem Gradienten der Winkelgeschwindigkeitswellenform umfassen;
Bestimmen (2) der den Aktionsdaten der ersten Aktion entsprechenden Tragefestigkeit der tragbaren Vorrichtung, das eine Bestimmung der Tragefestigkeit der tragbaren Vorrichtung basierend auf einer Entsprechung zwischen voreingestellten Daten des ersten Aktionsknotens und der Tragefestigkeit und den ersten dem ersten Aktionsknoten entsprechenden Merkmalsdaten umfasst; und
Senden (3) eines Detektionsergebnisses der Tragefestigkeit der tragbaren Vorrichtung an eine nicht tragbare Vorrichtung; und
die nicht tragbare Vorrichtung, konfiguriert zum:
Empfangen (4) des Detektionsergebnisses der Tragefestigkeit der tragbaren Vorrichtung und
Erzeugen (4) von Aufforderungsinformationen basierend auf dem Detektionsergebnis der Tragefestigkeit der tragbaren Vorrichtung, wobei die Aufforderungsinformationen zum Benachrichtigen des Benutzers über das Detektionsergebnis der Tragefestigkeit der tragbaren Vorrichtung dienen.

7. Computerlesbares Speichermedium, wobei das computerlesbare Speichermedium Computeranweisungen umfasst und, wenn die Computeranweisungen auf einem System nach Anspruch 6 laufen, das System befähigt wird, das Verfahren zum Detektieren der Tragefestigkeit einer tragbaren Vorrichtung nach einem der Ansprüche 1 bis 5 durchzuführen.

## Revendications

1. Procédé de détection d'un serrement de port d'un dispositif portable, dans lequel le procédé comprend :
la collecte (1), par le dispositif portable, de données de mouvement d'un utilisateur lorsqu'il est détecté que le dispositif portable est dans un état porté, dans lequel les données de mouvement de l'utilisateur comprennent une accélération et/ou une vitesse angulaire ;
l'identification (2), par le dispositif portable, de données d'action d'une première action dans les données de mouvement de l'utilisateur, dans lequel la première action est une action de l'utilisateur dans un processus de mouvement, la première action correspond à une position où le dispositif portable est porté, et lorsque le dispositif portable est porté sur un bras ou un poignet de l'utilisateur, la première action comprend une action de balancement de bras, et lorsque le dispositif portable est porté sur une cheville ou un pied ou une chaussure de l'utilisateur, la première action comprend une action de saut ; et
l'identification (2), par le dispositif portable, de données d'action d'un premier nœud d'action dans les données d'action de la première action, dans lequel, lorsque la première action comprend une action de balancement de bras, le premier nœud d'action comporte : un bras se balance sur un côté avant d'un corps au niveau d'une position spécifiée et/ou le bras se balance sur un côté arrière du corps au niveau d'une position spécifiée, et lorsque la première action comprend une action de saut,
le premier nœud d'action comporte : un pied quitte le sol et/ou le pied touche le sol ; la détermination (2), par le dispositif portable, de premières données de caractéristique correspondant au premier nœud d'action sur la base des données d'action du premier nœud d'action, dans lequel les premières données de caractéristique comprennent au moins l'une parmi une quantité de crête d'onde d'une forme d'onde d'accélération, une quantité de crête d'onde d'une forme d'onde de vitesse angulaire, une quantité de creux d'onde de la forme d'onde d'accélération, une quantité de creux d'onde de la forme d'onde de vitesse angulaire, une valeur de crête de la forme d'onde d'accélération, une valeur de crête de la forme d'onde de vitesse angulaire, une valeur moyenne d'accélération, une valeur moyenne de vitesse angulaire, un gradient de la forme d'onde d'accélération et un gradient de la forme d'onde de vitesse angulaire ;
la détermination (2), par le dispositif portable, du serrement de port du dispositif portable correspondant aux données d'action de la première action, comprenant la détermination du serrement de port du dispositif portable sur la base d'une correspondance entre des données prédéfinies du premier nœud d'action et le serrement de port et les premières données de caractéristique correspondant au premier nœud d'action ;
l'envoi (3), par le dispositif portable, d'un résultat de détection du serrement de port du dispositif portable à un dispositif non portable ;
la réception (4), par le dispositif non portable, du résultat de détection du serrement de port du dispositif portable ; et
la génération (4), par le dispositif non portable, d'informations d'invite sur la base du résultat de détection du serrement de port du dispositif portable afin de notifier à l'utilisateur le résultat de détection du serrement de port du dispositif portable.

2. Procédé selon la revendication 1, dans lequel la détermination du serrement de port du dispositif portable correspondant aux données d'action de la première action comprend :
la détermination, sur la base des données d'action de la première action et d'un modèle de serrement de port, du serrement de port du dispositif portable correspondant aux données d'action de la première action, dans lequel
les données d'action de la première action sont utilisées en tant qu'entrée du modèle de serrement de port, et le serrement de port du dispositif portable est utilisé en tant que sortie du modèle de serrement de port.

3. Procédé selon l'une quelconque des revendications 1 à 2, dans lequel les informations d'invite présentent un résultat de détection du serrement de port du dispositif portable sous une forme de présentation prédéfinie, dans lequel la forme de présentation prédéfinie comprend une ou une combinaison de ce qui suit : une forme de présentation de son, une forme de présentation de texte, une forme de présentation de vibration et une forme de présentation de lumière.

4. Procédé selon l'une quelconque des revendications 1 à 3, dans lequel le serrement de port du dispositif portable comprend : le dispositif portable est porté normalement ; le dispositif portable est porté de manière lâche ; et le dispositif portable est porté de manière serrée.

5. Procédé selon l'une quelconque des revendications 1 à 4, comprenant en outre :
la détermination d'un serrement de port du dispositif portable à chaque instant pendant la première durée ;
la détermination d'une seconde durée pendant laquelle le dispositif portable est porté de manière lâche sur la base du serrement de port du dispositif portable à chaque instant pendant la première durée ; et
le calcul d'une seconde énergie de mouvement de l'utilisateur pendant la première durée sur la base de la première durée, de la seconde durée et d'une première énergie de mouvement calculées par le dispositif portable, dans lequel la première énergie de mouvement est obtenue par calcul lorsqu'un état de port du dispositif portable pendant la première durée est normal ou serré.

6. Système de détection de serrement de port d'un dispositif portable, dans lequel le système comprend :
le dispositif portable, configuré pour :
collecter (1) des données de mouvement d'un utilisateur lorsqu'il est détecté que le dispositif portable est dans un état porté, dans lequel les données de mouvement de l'utilisateur peuvent comprendre une accélération et/ou une vitesse angulaire ;
identifier (2) des données d'action d'une première action dans les données de mouvement de l'utilisateur, dans lequel la première action est une action de l'utilisateur dans un processus de mouvement, la première action correspond à une position où le dispositif portable est porté, et lorsque le dispositif portable est porté sur un bras ou un poignet de l'utilisateur, la première action comprend une action de balancement de bras, et lorsque le dispositif portable est porté sur une cheville ou une chaussure de l'utilisateur, la première action comprend une action de saut ;
identifier (2) des données d'action d'un premier nœud d'action dans les données d'action de la première action, dans lequel, lorsque la première action comprend une action de balancement de bras, le premier nœud d'action comporte : un bras se balance sur un côté avant d'un corps au niveau d'une position spécifiée et/ou le bras se balance sur un côté arrière du corps au niveau d'une position spécifiée, et lorsque la première action comprend une action de saut, le premier nœud d'action comporte : un pied quitte le sol et/ou le pied touche le sol ;
déterminer (2) de premières données de caractéristique correspondant au premier nœud d'action sur la base des données d'action du premier nœud d'action, dans lequel les premières données de caractéristique comprennent au moins l'une parmi une quantité de crête d'onde d'une forme d'onde d'accélération, une quantité de crête d'onde d'une forme d'onde de vitesse angulaire, une quantité de creux d'onde de la forme d'onde d'accélération, une quantité de creux d'onde de la forme d'onde de vitesse angulaire, une valeur de crête de la forme d'onde d'accélération, une valeur de crête de la forme d'onde de vitesse angulaire, une valeur moyenne d'accélération, une valeur moyenne de vitesse angulaire, un gradient de la forme d'onde d'accélération et un gradient de la forme d'onde de vitesse angulaire déterminer (2) le serrement de port du dispositif portable correspondant aux données d'action de la première action, comprenant une détermination du serrement de port du dispositif portable sur la base d'une correspondance entre des données prédéfinies du premier nœud d'action et le serrement de port et les premières données de caractéristique correspondant au premier nœud d'action ; et
envoyer (3) un résultat de détection du serrement de port du dispositif portable à un dispositif non portable ; et
le dispositif non portable, configuré pour :
recevoir (4) le résultat de détection du serrement de port du dispositif portable, et
générer (4) des informations d'invite sur la base du résultat de détection du serrement de port du dispositif portable, dans lequel les informations d'invite servent à notifier à l'utilisateur le résultat de détection du serrement de port du dispositif portable.

7. Support de stockage lisible par ordinateur, dans lequel le support de stockage lisible par ordinateur comprend des instructions informatiques, et lorsque les instructions informatiques sont exécutées sur un système selon la revendication 6, le système est activé pour effectuer le procédé de détection de serrement de port d'un dispositif portable selon l'une quelconque des revendications 1 à 5.
